(19) Europäisches Patentamt
European Patent Office
Office européen des brevets

(11) Numéro de publication : **0 514 276 A1**

# (12) DEMANDE DE BREVET EUROPEEN

(21) Numéro de dépôt : **92401332.9**

(22) Date de dépôt : **15.05.92**

(51) Int. Cl.[5] : **C07D 495/04,**
// (C07D495/04, 335:00, 209:00)

(30) Priorité : **17.05.91 FR 9106038**

(43) Date de publication de la demande :
**19.11.92 Bulletin 92/47**

(84) Etats contractants désignés :
**PT**

(71) Demandeur : **RHONE-POULENC RORER SA**
**20, avenue Raymond Aron**
**F-92160 Antony (FR)**

(72) Inventeur : **Achard, Daniel**
**26 rue Adrien Tessier**
**F-94320 Thiais (FR)**
Inventeur : **Moutonnier, Claude**
**3 rue Auguste Rodin**
**F-92350 Le Plessis Robinson (FR)**
Inventeur : **Peyronel, Jean-François**
**6 Parc d'Ardenay**
**F-91120 Palaiseau (FR)**
Inventeur : **Tabart, Michel**
**Tour Athènes, 75 rue du Javelot**
**F-75013 Paris (FR)**
Inventeur : **Truchon, Alain**
**73 rue Henri Gorjus**
**F-69004 Lyon (FR)**

(74) Mandataire : **Savina, Jacques et al**
**Rhône-Poulenc Rorer S.A. Direction Brevets**
**(t144) 20 avenue Raymond Aron**
**F-92165 Antony Cédex (FR)**

## (54) Nouveaux dérivés du thiopyranopyrrole et leur préparation.

(57) Nouveaux dérivés de thiopyranopyrrole de formule générale (I) dans laquelle R est hydrogène, allyle ou un radical de structure :

$$-C\ R_aR_bR_c$$

dans laquelle $R_a$ et $R_b$ sont des atomes d'hydrogène ou des radicaux phényle éventuellement substitués (par halogène, alcoyle, alcoyloxy ou nitro), et $R_c$ est défini comme $R_a$ et $R_b$ ou représente un radical alcoyle ou alcoyloxyalcoyle, l'un au moins de $R_a$, $R_b$ et $R_c$ étant un radical phényle substitué ou non, et n = 0 à 2, sous leurs formes stéréoisomères et leurs mélanges, éventuellement leurs sels lorsqu'ils existent et leur préparation.

Les nouveaux dérivés selon l'invention sont particulièrement intéressants comme intermédiaires de synthèse.

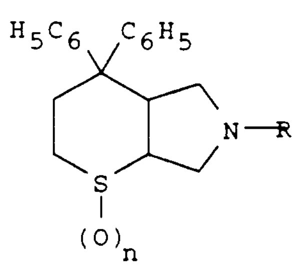

(I)

EP 0 514 276 A1

Jouve, 18, rue Saint-Denis, 75001 PARIS

La présente invention concerne de nouveaux dérivés de thiopyranopyrrole de formule générale :

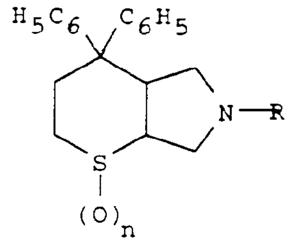

(I)

ainsi que leurs sels lorsqu'ils existent, utiles comme intermédiaires pour la préparation de dérivés du thiopyranopyrrole antagonistes des effets de la substance P.

Dans le brevet américain 4 042 707 avaient été décrits des produits dérivés de l'isoindole de formule générale:

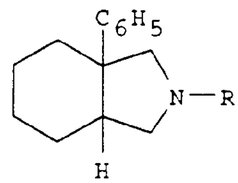

ayant une activité opiacée.

Ces produits n'ont pas d'activité vis à vis de la substance P et ne sont pas non plus utilisés pour la synthèse de tels produits.

Dans la demande européenne 0068822 ont été décrits des herbicides répondant à la formule générale :

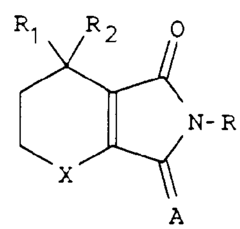

dans laquelle X peut être un atome de soufre, $R_1$ et $R_2$ sont hydrogène ou alcoyle et R est phényle substitué.

Jusqu'à présent, malgré les recherches mises en oeuvre et malgré l'intérêt suscité (M.R. Hanley, TINS,(5) 139 (1982)], il n'avait pratiquement pas été découvert de produit agissant spécifiquement sur la substance P et ayant une structure non peptidique, c'est pourquoi les dérivés de thiopyranopyrrole de formule générale (I) présentent un intérêt considérable dans la mesure où ils permettent l'accès à de tels produits.

Dans la formule générale (I), le symbole R représente un atome d'hydrogène, un radical allyle ou un radical de structure :

$$- C\ R_aR_bR_c \quad (Ia)$$

dans laquelle $R_a$ et $R_b$ sont des atomes d'hydrogène ou des radicaux phényle éventuellement substitués (par un atome d'halogène, un radical alcoyle, alcoyloxy ou nitro), et $R_c$ est défini comme $R_a$ et $R_b$ ou représente un radical alcoyle ou alcoyloxyalcoyle, l'un au moins de $R_a$, $R_b$ et $R_c$ étant un radical phényle substitué ou non, et le symbole n représente un nombre entier de 0 à 2.

Il est entendu que les radicaux alcoyle cités ci-dessus contiennent 1 à 4 atomes de carbone en chaîne droite ou ramifiée.

Les produits de formule générale (I) présentent différentes formes stéréoisomères; il est entendu que les dérivés de thiopyranopyrrole de forme (4aR,7aR) ou de forme (4aS,7aS) à l'état pur, ou sous forme de mélange des formes cis (4aRS,7aRS), entrent dans le cadre de la présente invention.

De plus, les produits de formule générale (I) pour lesquels n=1 présentent également des stéréoisomères axiaux ou équatoriaux au niveau du S-oxyde. Il est entendu que les dérivés R et S en position -1 ainsi que leurs mélanges entrent aussi dans le cadre de la présente invention.

Selon l'invention, le dérivé de thiopyranopyrrole de formule générale (I) peut être obtenu par traitement du

dérivé de formule générale :

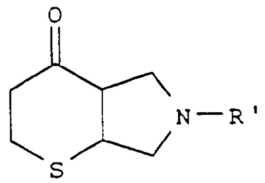

(II)

dans laquelle R′ est défini comme R à l'exception de représenter l'atome d'hydrogène, successivement par un halogénure de phényl-magnésium, puis par le benzène en présence de tétrachlorure de zirconium, suivi éventuellement de l'élimination du radical protecteur R′ si l'on veut obtenir un produit pour lequel R est un atome d'hydrogène, et/ou suivi le cas échéant de l'oxydation du produit obtenu, pour obtenir un dérivé de thiopyranopyrrole pour lequel n=1 ou 2.

Le traitement du dérivé de thiopyranopyrrole de formule générale (II) s'effectue selon les méthodes habituelles. Le traitement par un halogénure de phényl magnésium s'effectue avantageusement au moyen du bromure de phényl magnésium, dans un éther (éther éthylique par exemple) à une température comprise entre 20°C et la température de reflux du mélange réactionnel. Le traitement par le benzène en présence de tétrachlorure de zirconium s'effectue à une température comprise entre 20°C et la température de reflux du mélange réactionnel.

Le cas échéant lorsque l'on veut éliminer le radical R′, on opère par toute méthode connue qui n'affecte pas le reste de la molécule.

Notamment lorsque R′ est autre qu'un radical allyle, le groupement R′ peut être éliminé par hydrogénation catalytique en présence de palladium. Généralement, la réaction s'effectue en milieu acide, dans un solvant tel qu'un alcool (méthanol, éthanol), dans l'eau ou directement dans l'acide acétique ou l'acide formique, à une température comprise entre 20 et 60°C.

Lorsque R′ est un radical benzhydryle ou trityle, l'élimination peut être effectuée par traitement en milieu acide, en opérant à une température comprise entre O°C et la température de reflux du mélange réactionnel, dans un alcool, dans un éther, dans l'eau ou directement dans l'acide acétique, l'acide formique ou l'acide trifluoroacétique.

Le groupement R′ peut être également éliminé en faisant agir le chloroformiate de vinyle, le chloroformiate de chloro-1 éthyle ou le chloroformiate de phényle, en passant intermédiairement par un produit de formule générale :

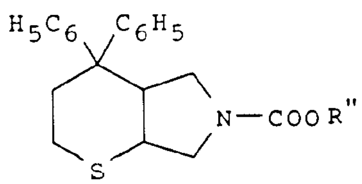

(III)

dans laquelle R″ est un radical vinyle, chloro-1 éthyle ou phényle, puis par élimination du radical R″ par traitement acide. L'action du chloroformiate s'effectue généralement dans un solvant organique tel qu'un solvant chloré (dichlorométhane, dichloréthane, chloroforme par exemple), un éther (tétrahydrofuranne, dioxanne par exemple) ou une cétone (acétone par exemple) ou dans un mélange de ces solvants, en opérant à une température comprise entre 20°C et la température de reflux du mélange réactionnel.

L'élimination du radical R″ est effectuée par traitement en milieu acide par exemple par l'acide trifluoroacétique, formique, méthanesulfonique, p.toluènesulfonique, chlorhydrique ou bromhydrique dans un solvant tel qu'un alcool, un éther, un ester, un nitrile, un mélange de ces solvants ou dans l'eau, à une température comprise entre 0°C et la température de reflux du mélange réactionnel.

Dans les conditions d'élimination des radicaux R″ citées précédemment, le dérivé de thiopyranopyrrole de formule générale (I) est obtenu directement à l'état de sel de l'acide employé.

Lorsque l'on veut obtenir un dérivé de thiopyranopyrrole de formule générale (I) pour lequel n égale 1 ou 2, la réaction d'oxydation s'effectue par toute méthode connue pour l'oxydation de sulfures en sulfoxydes ou en sulfones, qui n'altère pas le reste de la molécule, sur le produit pour lequel la fonction amine est protégée. Par exemple, on opère par action d'un peracide organique (acide percarboxylique ou persulfonique, notamment l'acide perbenzoïque, l'acide chloro-3 perbenzoïque, l'acide nitro-4 perbenzoïque, l'acide peracétique, l'acide

pertrifluoracétique, l'acide performique, l'acide permaléique, l'acide monoperphtalique, l'acide percamphorique ou pertoluènesulfonique) ou les peracides minéraux (par exemple l'acide periodique ou persulfurique). La réaction s'effectue avantageusement dans un solvant chloré (chlorure de méthylène) à une température comprise entre 0 et 25°C. Il est également possible d'opérer au moyen de tertiobutylhydroperoxyde en présence de tétraisopropylate de titane.

Lorsque l'on veut obtenir un produit de formule générale (I) pour lequel n=2, on opère au moyen de 2 équivalents d'agent oxydant.

Le cas échéant, le choix, la mise en place et l'élimination du radical protecteur d'amino s'effectuent selon les méthodes habituelles qui n'affectent pas le reste de la molécule; notamment on opère selon les méthodes décrites par T.W. Greene, Protective Groups in Organic Synthesis, A. Wiley - Interscience Publication (1981), ou par Mc Omie, Protective Groups in Organic Chemistry, Plenum Press (1973).

Il est également avantageux d'opérer à partir du produit de formule générale (I) pour lequel n=0 sous forme de sel avec un acide minéral (chlorhydrate, sulfate par exemple).

Dans la pratique, il est entendu que pour préparer un produit de formule générale (I) pour lequel n=1 ou 2 et pour lequel R est un atome d'hydrogène, il est avantageux d'effectuer l'oxydation préalablement à l'élimination du radical protecteur R'.

Selon l'invention, le dérivé de thiopyranopyrrole de formule générale (I) pour lequel n = 2 peut également être obtenu à partir de dihydro-3,4 diphényl-4,4 di-oxyde-1,1 2H-thiapyranne de formule :

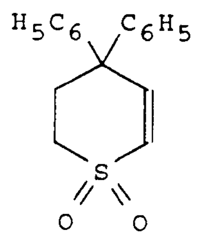

(IV)

par réaction de cycloaddition avec un dérivé silylé de formule générale :

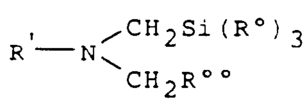

(V)

dans laquelle R' est défini comme précédemment, $(R°)_3$ représente des radicaux alcoyle ou des radicaux alcoyle et phényle et R°° représente un radical alcoyloxy, cyano ou phénylthio, suivie éventuellement de l'élimination du radical protecteur R' dans les conditions décrites précédemment, si l'on veut obtenir un dérivé de formule générale (I) pour lequel R est un atome d'hydrogène.

La réaction de cycloaddition s'effectue en présence d'une quantité catalytique d'un acide choisi parmi l'acide trifluoroacétique, l'acide acétique, l'acide méthanesulfonique ou les acides cités dans les références mentionnées ci-dessous, dans un solvant organique tel qu'un solvant chloré (dichlorométhane, dichloréthane par exemple), dans un hydrocarbure aromatique, dans un nitrile (acétonitrile) ou dans un éther, à une température comprise entre O°C et la température de reflux du mélange réactionnel.

Le dérivé de thiopyranopyrrole de formule générale (II) peut être obtenu par réaction de cycloaddition par action d'un dérivé silylé de formule générale (V) sur la déhydrothiapyranone-4 de formule :

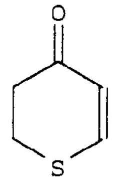

(VI)

dans des conditions identiques à celles décrites précédemment pour la réaction de cycloaddition de ce produit sur la sulfone de formule (IV).

Le dérivé silylé de formule générale (V) peut être obtenu selon les méthodes décrites par :

- Y. Terao et coll., Chem. Pharm. Bull., 33, 2762 (1985);
- A. Hosomi et coll., Chem. Lett., 1117 (1984) ;
- A. Padwa et coll., Chem. Ber., 119, 813 (1986) ou
- Tetrahedron, 41, 3529 (1985).

Le dihydro-3,4 diphényl-4,4 dioxyde-1,1 2H-thiapyranne de formule (IV) peut être obtenu par oxydation successive du dihydro-3,4 diphényl-4,4 2H-thiapyranne et du dihydro-3,4 diphényl-4,4 oxyde-1 2H-thiapyranne de formules :

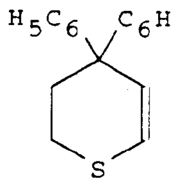

(VII)

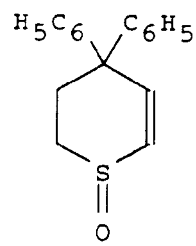

(VIII)

La réaction d'oxydation s'effectue dans les conditions décrites précédemment pour la préparation des produits de formule générale (I). Il n'est pas indispensable d'isoler le S-oxyde de formule générale (VIII) pour l'oxyder en sulfone.

Le dihydro-3,4 diphényl-4,4 2H-thiapyranne de formule générale (VII) peut être préparé selon ou par analogie avec la méthode décrite ci-après à l'exemple 5.

Il est entendu que les dérivés de thiopyranopyrrole de formule générale (I), (II) et (III) présentent plusieurs formes stéréoisomères. La séparation des stéréoisomères (4aR, 7aR) ou (4aS,7aS) est mise en oeuvre avantageusement au niveau du dérivé de formule générale (I).

La séparation des stéréoisomères s'effectue selon toute méthode connue et compatible avec la molécule. A titre d'exemple, la séparation peut être effectuée par préparation d'un sel optiquement actif, par action de l'acide L(+) ou D(-) mandélique, ou de l'acide dibenzoyltartrique, puis séparation des isomères par cristallisation. L'isomère recherché est libéré de son sel en milieu basique.

La séparation des isomères axial et équatorial peut être effectuée par chromatographie ou cristallisation.

Selon l'invention, les nouveaux dérivés du thiopyranopyrrole de formule générale (I) sont utiles pour la préparation de dérivés qui antagonisent les effets de la substance P et répondent à la formule générale :

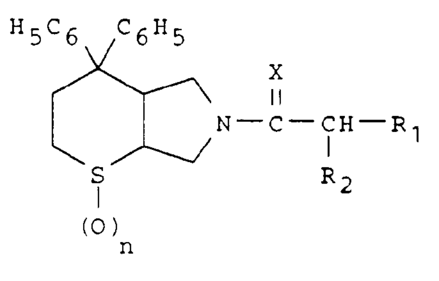

(IX)

dans laquelle :

- n est défini comme précédemment,
- le symbole X représente un atome d'oxygène ou un radical NH,
- le symbole $R_1$ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitués [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy ou hydroxyalcoyle)], ou substitué par des radicaux amino, alcoylamino, dialcoylamino dont les parties alcoyle peuvent former avec l'atome d'azote auquel elles sont rattachées, un hétérocycle tel que défini cidessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre, et

- le symbole $R_2$ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino,

les radicaux alcoyle ou acyle cités ci-dessus contenant 1 à 4 atomes de carbone en chaîne droite ou ramifiée.

Dans la formule générale (IX) ci-dessus, lorsque $R_1$ contient un atome d'halogène, ce dernier peut être choisi parmi le chlore, le brome, le fluor ou l'iode;

lorsque $R_1$ représente un radical hétérocyclyle mono ou polycyclique, saturé ou insaturé, à titre d'exemple il peut être choisi parmi thiényle, furyle, pyridyle, dithiinyle, indolyle, isoindolyle, thiazolyle, isothiazolyle, oxazolyle, imidazolyle, pyrrolyle, triazolyle, thiadiazolyle, quinolyle, isoquinolyle, ou naphtyridinyle;

lorsque $R_1$ représente phényle substitué par une chaîne portant un hétérocycle, ce dernier peut être choisi parmi pyrrolidinyle, morpholino, pipéridinyle, tétrahydropyridinyle, pipérazinyle ou thio-morpholino;

de plus, lorsque le symbole $R_2$ est autre que l'atome d'hydrogène, la chaîne substituée sur le thiopyranopyrrole présente un centre chiral, il est entendu que les formes stéréoisomères et leurs mélanges entrent aussi dans le cadre de la formule générale (IX).

Les dérivés de thiopyranopyrrole de formule générale (IX) peuvent être obtenus par action de l'acide de formule générale :

$$R_1 - \underset{\displaystyle R_2}{\underset{|}{CH}} - COOH \qquad (X)$$

ou d'un dérivé réactif de cet acide, dans lequel $R_1$ et $R_2$ sont définis comme précédemment, sur un dérivé de thiopyranopyrrole de formule générale (I) pour lequel R est un atome d'hydrogène et n est défini comme précédemment, suivie le cas échéant de la transformation de l'amide obtenu en une amidine pour laquelle X représente un radical NH.

Il est entendu que, les radicaux amino, alcoylamino ou carboxy contenus dans $R_1$ et/ou $R_2$ sont de préférence préalablement protégés.

La protection s'effectue par tout groupement compatible, dont la mise en place et l'élimination n'affectent pas le reste de la molécule. Notamment, selon les méthodes citées précédemment.

A titre d'exemple,

- les groupements amino ou alcoylamino peuvent être protégés par des radicaux méthoxycarbonyle, éthoxycarbonyle, t.butoxycarbonyle, allyloxycarbonyle, vinyloxycarbonyle, trichloréthoxycarbonyle, trichloracétyle, trifluoracétyle, chloracétyle, trityle, benzhydryle, benzyle, allyle, formyle, acétyle, benzyloxycarbonyle ou ses dérivés substitués;
- les groupements acides peuvent être protégés par des radicaux méthyle, éthyle, t.butyle, benzyle, benzyle substitué ou benzhydryle.

De plus, lorsque $R_2$ représente un radical hydroxy, il est préférable de protéger préalablement ce radical. La protection s'effectue par exemple par un radical acétoxy, trialcoylsilyle, benzyle, ou sous forme d'un carbonate par un radical -COORa dans lequel Ra est un radical alcoyle ou benzyle.

Lorsque l'on effectue la condensation d'un dérivé réactif de l'acide de formule générale (X), on opère avantageusement au moyen du chlorure d'acide, de l'anhydride, d'un anhydride mixte ou d'un ester réactif dans lequel le reste de l'ester est par exemple un radical succinimido, benzotriazolyl-1, nitro-4 phényle, dinitro-2,4 phényle, pentachlorophényle ou phtalimido ou un dérivé.

La réaction s'effectue généralement à une température comprise entre -40 et +40°C, dans un solvant organique tel qu'un solvant chloré (dichlorométhane, dichloréthane, chloroforme par exemple), un éther (tétrahydrofuranne, dioxanne par exemple), un ester (acétate d'éthyle par exemple), un amide (diméthylacétamide, diméthylformamide par exemple), ou une cétone (acétone par exemple) ou dans un mélange de ces solvants, en présence d'un accepteur d'acide tel qu'une base organique azotée comme par exemple la pyridine, la diméthylaminopyridine, la N-méthylmorpholine ou une trialcoylamine (notamment triéthylamine) ou tel qu'un époxyde (oxyde de propylène par exemple). Il est également possible d'opérer en présence d'un agent de condensation tel qu'un carbodiimide, [par exemple dicyclohexylcarbodiimide ou (diméthylamino-3 propyl)-1 éthyl-3 carbodiimide), le NN'-carbonyldiimidazole ou l'éthoxy-2 éthoxycarbonyl-1 dihydro-1,2 quinoléine ou bien en milieu hydroorganique, en présence d'un agent alcalin de condensation comme le bicarbonate de sodium, puis on transforme le cas échéant l'amide obtenu en une amidine telle que définie précédemment.

La transformation de l'amide de formule générale (IX) en une amidine pour laquelle X est un radical NH s'effectue en préparant le dérivé de formule générale :

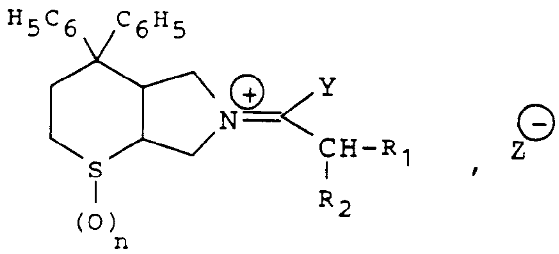

(XI)

dans laquelle $R_1$, $R_2$ et n sont définis comme précédemment, Y représente un atome de chlore, un radical méthoxy ou éthoxy et $Z^-$ représente un ion chlorure, tétrafluoroborate, fluorosulfonate, trifluorométhylsulfonate, méthylsulfate, ou éthylsulfate, puis en faisant agir l'ammoniac sur le dérivé de formule générale (XI).

La préparation du dérivé de formule générale (XI) dans laquelle Y est un atome de chlore ou un radical méthoxy ou éthoxy s'effectue par action d'un réactif tel que le phosgène, l'oxychlorure de phosphore, le pentachlorure de phosphore, le chlorure de thionyle, le chlorure d'oxalyle, le chloroformate de trichlorométhyle, le tétrafluoroborate de triéthyl (ou de triméthyl) oxonium, le triflate de méthyle (ou d'éthyle), le fluorosulfonate de méthyle (ou d'éthyle) ou le sulfate de méthyle (ou d'éthyle). La réaction s'effectue dans un solvant chloré (dichlorométhane, dichloréthane par exemple) ou dans un hydrocarbure aromatique (toluène par exemple), à une température comprise entre O°C et la température de reflux du mélange réactionnel. L'action de l'ammmoniac sur le dérivé de formule générale (XI) s'effectue dans un solvant organique anhydre tel qu'un solvant chloré (dichlorométhane, dichloréthane par exemple), dans un mélange alcool-solvant chloré, dans un éther (tétrahydrofurane par exemple), dans un ester (par exemple acétate d'éthyle), dans un solvant aromatique (toluène par exemple) ou dans un mélange de ces solvants, à une température comprise entre -2O°C et la température de reflux du mélange réactionnel.

Il n'est pas indispensable d'avoir isolé le dérivé de formule générale (XI) pour le mettre en oeuvre dans cette réaction.

Les acides de formule générale (X) peuvent être préparés selon les méthodes décrites ci-après dans les exemples, ou par analogie avec ces méthodes.

Les dérivés de thiopyranopyrrole de formule générale (IX) pour lesquels X est un radical NH peuvent également être obtenus à partir du dérivé de thiopyranopyrrole de formule générale (I) pour lequel R est un atome d'hydrogène, par action d'un produit de formule générale :

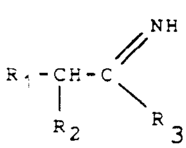

(XII)

éventuellement à l'état de sel, dans laquelle $R_1$ et $R_2$ sont définis comme précédemment et $R_3$ représente un radical alcoyloxy contenant 1 à 4 atomes de carbone en chaîne droite ou ramifiée ou un radical méthylthio, éthylthio, benzylthio ou alcoyloxycarbonylméthylthio.

La réaction est mise en oeuvre au moyen du dérivé de formule générale (XII) éventuellement préparé in situ, dans un solvant organique tel qu'un solvant chloré (dichlorométhane, dichloréthane par exemple), un éther (tétrahydrofuranne par exemple), un hydrocarbure aromatique (toluène par exemple) ou un nitrile par exemple l'acétonitrile à une température comprise entre O°C et la température de reflux du mélange réactionnel.

Il est entendu que, dans l'éventualité où les radicaux $R_1$ et/ou $R_2$ du produit de formule générale (XII) portent des substituants pouvant interférer avec la réaction, ces derniers doivent être préalablement protégés.

Les nouveaux dérivés de thiopyranopyrrole de formule générale (I) ainsi que les dérivés de formule générale (IX) auxquels ils conduisent peuvent être purifiés le cas échéant par des méthodes physiques telles que la cristallisation ou la chromatographie.

Le cas échéant, les nouveaux dérivés de formule générale (I) ou les dérivés de formule générale (IX) pour lesquels les symboles $R_1$ et/ou $R_2$ contiennent des substituants amino ou alcoylamino et/ou X représente un

radical NH, peuvent être transformés en sels d'addition avec les acides. Comme exemples de sels d'addition avec des acides , peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, p.toluènesulfonates, iséthionates, ou avec des dérivés de substitution de ces composés).

La substance P est connue pour être impliquée dans un certain nombre de domaines pathologiques :

- Agonists and antagonists of substance P, A.S. Dutta Drugs of the futur, 12 (8), 782 (1987);
- Substance P and pain : an updating, J.L. Henry, TINS, 3(4), 97 (1980);
- Substance P in inflammatory reactions and pain, S. Rosell, Actual. Chim. Ther., 12ème série, 249 (1985)
- Effects of Neuropeptides on Production of Inflammatory Cytokines by Human Monocytes, M. Lotz et coll., Science, 241, 1218 (1988).
- Neuropeptides and the pathogenesis of allergy, Allergy, 42, 1 à 11 (1987);
- Substance P in Human Essential Hypertension, J. Cardiocascular Pharmacology, 10 (suppl. 12), 5172 (1987).

Les dérivés de thiopyranopyrrole de formule générale (IX) qui antagonisent les effets de la substance P peuvent trouver une application dans les domaines de l'analgésie, de l'inflammation de l'asthme, des allergies, sur le système nerveux central, sur le système cardiovasculaire, comme antispasmodique, ou sur le système immunitaire, ainsi que dans le domaine de la stimulation des secrétions lachrymales.

En effet, ces produits manifestent une affinité pour les récepteurs à substance P à des doses comprises entre 10 et 2000 nM selon la technique décrite par C.M. Lee et coll., Mol. Pharmacol., 23, 563-69 (1983).

Il a de plus été démontré qu'il s'agit d'un effet antagoniste de la substance P, au moyen de différents produits. Dans la technique décrite par S. Rosell et coll., Substance P, Ed. by US Von Euler and B. Pernow, Raven Press, New-York (1977), pages 83 à 88, les produits étudiés se sont montrés actifs à des doses comprises entre 20 et 2000 nM.

Par ailleurs, les dérivés de thiopyranopyrrole selon la présente invention ne présentent pas de toxicité, ils se sont montrés atoxiques chez la souris par voie sous cutanée à la dose de 40 mg/kg ou par voie orale à la dose de 100 mg/kg.

D'un intérêt particulier sont les produits suivants :

- Le diphényl-4,4 perhydrothiopyrano[2,3-c]pyrrole ;
- Le diphényl-4,4 oxyde-1 perhydrothiopyrano[2,3-c]pyrrole ;
- Le diphényl-4,4 perhydrothiopyrano[2,3-c]pyrrole dioxyde-1,1 ;
- Le diphényl-4,4 vinyloxycarbonyl-6 perhydrothiopyrano[2,3-c]pyrrole ;
- Le diphényl-4,4 oxide-1 tertiobutyloxycarbonyl-6 perhydrothiopyrano[2,3-c]pyrrole ;

ainsi que leurs sels, leurs formes stéréoisomères et leurs mélanges.

Les exemples suivants donnés à titre non limitatif illustrent la présente invention.

Dans les exemples qui suivent, il est entendu que, sauf mention spéciale, les spectres de RMN du proton ont été faits à 250 MHz dans le diméthylsulfoxyde; les déplacements chimiques sont exprimés en ppm.

Exemple 1

On traite 4,35 g de diphényl-4,4 vinyloxycarbonyl-6 perhydrothiopyrano [2,3-c]pyrrole-(4aRS,7aRS) par 30 $cm^3$ d'une solution 5,7 N d'acide chlorhydrique dans le dioxane sec pendant 30 minutes à 20°C. La solution est concentrée à sec sous pression réduite (2,7 kPa), le résidu est repris par 150 $cm^3$ d'éthanol, la solution résultante est portée au reflux pendant 30 minutes, puis est concentrée à sec. Le solide obtenu est lavé par 50 $cm^3$ d'éther éthylique, essoré, séché. On obtient 3,64 g de chlorhydrate de diphényl-4,4 perhydrothiopyrano[2,3-c]pyrrole-(4aRS,7aRS), sous la forme d'un solide blanc.

Spectre infra-rouge (KBr, bandes caractéristiques, $cm^{-1}$): 3060, 3030, 3000, 2250, 1600, 1495, 1580, 1450, 755, 710, 700.

Spectre RMN du proton (DMSO-$d_6$,signaux principaux): 2,2 à 2,9 (mt, 4H, $CH_2$ en 2 et $CH_2$ en 3); 2,4 et 3,3 (2mt, 2H, $CH_2$ en 5); 3,08 (d, J=12,5, 1H, 1H en 7); 3,7 (mt, 1H, H en 4a); 4,16 (t, J=5, 1H, H en 7a); 7,1 à 7,5 (mt, 10 H, aromatiques).

Le diphényl-4,4 vinyloxycarbonyl-6 perhydrothiopyrano[2,3-c]pyrrole-(4aRS,7aRS) peut être préparé de la manière suivante:

A 6,2 g de benzyl-6 diphényl-4,4 perhydrothiopyrano[2,3-c]pyrrole-(4aRS,7aRS), dans 50 $cm^3$ de dichloro-1,2 éthane, on ajoute 1,72 $cm^3$ de chloroformiate de vinyle. Le mélange est porté au reflux pendant 15 minutes puis concentré à sec sous pression réduite (2,7 kPa). Le résidu est chromatographié sur une colonne de gel de silice (0,04 mm-0,06 mm, diamètre 25 cm), en éluant sous une pression de 0,6 bar d'azote, par un mélange de cyclohexane et d'acétate d'éthyle (90/10 en volumes), en recueillant des fractions de 60 $cm^3$. Les

fractions 5 à 16 sont réunies et concentrées à sec sous pression réduite (2,7 kPa), le résidu est trituré dans 70 $cm^3$ d'oxyde de diisopropyle, la suspension est filtrée, le solide essoré et séché. On obtient 4,35 g de diphényl-4,4 vinyloxycarbonyl-6 perhydrothiopyrano[2,3-c]pyrrole-(4aRS,7aRS), sous la forme d'un solide blanc fondant à 160°C.

Le benzyl-6 diphényl-4,4 perhydrothiopyrano[2,3-c]pyrrole-(4aRS,7aRS) peut être préparé de la façon suivante:

A une solution de 12,2 g d'hydroxy-4 phényl-4 benzyl-6 perhydrothiopyrano[2,3-c]pyrrole-(4RS,4aSR,7aRS) dans 180 $cm^3$ de benzène, on ajoute 43,7 g de tétrachlorure de zirconium. Le mélange réactionnel est porté au reflux pendant 1 heure puis amené à 20°C et dilué par 200 $cm^3$ de dichlorométhane. A la solution résultante refroidie, on ajoute 150 $cm^3$ d'une solution aqueuse d'hydroxyde de sodium 4N. La suspension obtenue est filtrée, le filtrat est décanté, la phase organique est lavée par 200 $cm^3$ de solution aqueuse saturée de chlorure de sodium, séchée sur sulfate de magnésium, concentrée à sec sous pression réduite (2,7 kPa). L'huile obtenue est chromatographiée sur une colonne de gel de silice (0,04 mm-0,06 mm, diamètre 5,2 cm, hauteur 39 cm), en éluant sous une pression d'azote de 0,6 bar par un mélange de cyclohexane et d'acétate d'éthyle (90/10 en volumes), en recueillant des fractions de 125 $cm^3$. Les fractions 19 à 32 sont réunies et concentrées à sec sous pression réduite (2,7 kPa). L'huile obtenue est cristallisée dans 200 $cm^3$ d'oxyde de diisopropyle, les cristaux sont essorés, séchés. On obtient 6,2 g de benzyl-6 diphényl-4,4 perhydrothiopyrano[2,3-c]pyrrole-(4aRS,7aRS), sous la forme de cristaux orangés, fondant à 130°C.

L'hydroxy-4 phényl-4 benzyl-6 perhydrothiopyrano[2,3-c]pyrrole-(4RS,4aSR,7aRS) peut être préparé de la façon suivante:

A une solution de bromure de phénylmagnésium préparée à partir de 19,8 $cm^3$ de bromobenzène et de 4,52 g de magnésium sec, dans 120 $cm^3$ d'éther éthylique anhydre, on ajoute en 30 minutes une solution de 21,15 g de benzyl-6 oxo-4 perhydrothiopyrano[2,3-c]pyrrole (4aRS,7aSR) dans 150 $cm^3$ d'éther éthylique anhydre. Le mélange réactionnel est agité à la température du reflux pendant 3 heures, puis pendant 20 heures à 20°C. Le mélange, additionné de 200 $cm^3$ d'éther éthylique est agité avec 600 $cm^3$ de solution aqueuse saturée de chlorure d'ammonium. La phase aqueuse est extraite par 200 $cm^3$ d'éther éthylique, les deux extraits éthérés réunis sont lavés deux fois par 300 $cm^3$ de solution aqueuse saturée de chlorure de sodium puis séchés sur sulfate de magnésium et concentrés à sec sous pression réduite (5,4 kPa) à 35°C. On obtient 12,2 g d'hydroxy-4 phényl-4 benzyl-6 perhydrothiopyrano[2,3-c]pyrrole-(4RS,4aSR,7aRS), sous la forme d'un solide blanc fondant à 137°C.

Le benzyl-6 oxo-4 perhydrothiopyrano[2,3-c]pyrrole-(4aRS,7aSR) peut être préparé de la manière suivante:

A une solution de 20 g de déhydrothiapyrannone-4 et de 54 $cm^3$ de N-butoxyméthyl N-triméthylsilylméthyl benzylamine dans 100 $cm^3$ de dichlorométhane anhydre, on ajoute 5 gouttes d'acide trifluoroacétique et agite pendant 4 heures, en maintenant la température à 20°C. Le mélange réactionnel est agité avec 5 g de carbonate de potassium, filtré, puis concentré à sec sous pression réduite (2,7 kPa).Le résidu huileux est chromatographié sur une colonne de gel de silice (0,04 mm-0,06 mm, diamètre 9,2 cm), en éluant sous une pression de 0,6 bar d'azote, par un mélange de cyclohexane et d'acétate d'éthyle (90/10 en volumes), puis par le mélange cyclohexane et acétate d'éthyle (75/25 en volumes), en recueillant des fractions de 250 $cm^3$. Les fractions 35 à 56 sont réunies et concentrées à sec sous pression réduite (2,7 kPa). On obtient 24 g de benzyl-6 oxo-4 perhydrothiopyrano[2,3-c]pyrrole-(4aRS,7aSR), sous la forme d'une huile jaune.

Spectre infra-rouge (Solution $CCl_4$, bandes caractéristiques, $cm^{-1}$): 3090, 3070, 3025, 2925, 2850, 2800, 2730, 1710, 1600, 1585, 1495, 1475, 1450, 700.

Spectre RMN du proton ($CDCl_3$,signaux principaux): 2,42 (dd, J=10 et 7, 1H, 1H en 7); 2,66 (mt, 2H, $CH_2$ en 5); 3,05 (mt, 1H, H en 4a); 3,1 (dd, J=10 et 7,5, 1H du $CH_2$ en 7); 3,61 (s, 2H, N-$CH_2$-Ar); 3,8 (dt, J=7,5 et 7, 1H, H en 7a); 7,15 à 7,35 (mt, 5H aromatiques).

Exemple 2

On traite 3,98 g de diphényl-4,4 oxyde-1 tertiobutyloxycarbonyl-6 perhydrothiopyrano[2,3-c]pyrrole (mélange d'isomères 1RS,4aSR,7aSR et 1RS,4aRS,7aRS) par 40 $cm^3$ d'un mélange d'acide chlorhydrique concentré (37% d'acide chlorhydrique) et de dioxane (1/2 en volumes) pendant 48 heures à 20°C. La solution est concentrée à sec à 40°C sous pression réduite (2,7 kPa). L'huile obtenue est reprise par 30 $cm^3$ de dichlorométhane, la solution est lavée par 60 $cm^3$ de solution aqueuse d'hydroxyde de sodium 2N, la phase aqueuse est extraite par 20 $cm^3$de dichlorométhane. Les extraits organiques sont réunis, séchés sur sulfate de magnésium, concentrés à sec à 40°C sous pression réduite (2,7 kPa). Le résidu est repris par de l'oxyde de diisopropyle, puis est concentré à sec à 40°C sous pression réduite (2,7 puis 0,13 kPa). On obtient 3,0 g de diphényl-4,4 oxyde-1 perhydrothiopyrano[2,3-c]pyrrole(1RS,4aRS,7aRS) sous la forme d'une meringue blanche.

Spectre infra-rouge (KBr, bandes caractéristiques, $cm^{-1}$): 3080, 3055, 3025, 2950, 2920, 2880, 2860, 1595, 1580, 1490, 1440, 1020, 760, 740, 700.

Spectre RMN du proton (DMSO-$d_6$ + $CF_3CCOD$, signaux principaux): 2,26 (t large, J=14, 1H, 1H en 3); 2,42 (dd, J=10 et 9, 1H, $CH_2$ en 5); 2,55 (dd large, J=14 et 4, 1H, 1H en 3); 3,68 (t, J=6, 1H, H en 7a); 3,82 (d, J=14, 1H, H en 7); 3,8 à 4 (mt, 1H, CH en 4a); 4,15 (dd, J=14 et 6, 1H, H en 7); 7,1 à 7,5 (mt, 10H aromatiques).

Le diphényl-4,4 oxyde-1 tertiobutyloxycarbonyl-6 perhydrothiopyrano [2,3-c]pyrrole (mélange d'isomères 1RS,4aSR,7aSR et 1RS,4aRS,7aRS) peut être préparé de la façon suivante:

A une solution refroidie à 0°C de 4,2 g de diphényl-4,4 tertiobutyloxycarbonyl-6 perhydrothiopyrano[2,3-c]pyrrole-(4aRS,7aRS) dans 30 $cm^3$ de dichlorométhane sec, on ajoute une solution de 2,3 g d'acide chloro-3 peroxybenzoïque (à 85%) dans 20 $cm^3$ de dichlorométhane. Après 1,5 heures d'agitation à 3°C et 1,5 heures à 20°C, le mélange réactionnel est lavé 2 fois par 100 $cm^3$ de solution aqueuse saturée de bicarbonate de sodium, puis par 100 $cm^3$ d'eau distillée, séché sur sulfate de magnésium, concentré à sec à 35°C sous pression réduite (2,7 kPa). Le résidu est cristallisé dans de l'acétate d'éthyle, les cristaux sont lavés par de l'acétate d'éthyle et de l'oxyde de diisopropyle, essorés, puis séchés sous pression réduite (2,7 kPa). On obtient 3,98 g de diphényl-4,4 oxyde-1 tertiobutyloxycarbonyl-6 perhydrothiopyrano[2,3-c]pyrrole-(mélange d'isomères 1RS,4aSR,7aSR et 1RS,4aRS,7aRS) sous la forme de cristaux blancs utilisés tel quel dans la réaction suivante.

Le diphényl-4,4 tertiobutyloxycarbonyl-6 perhydrothiopyrano[2,3-c] pyrrole-(4aRS,7aRS) peut être préparé de la manière suivante:

A une suspension de 4,0 g de chlorhydrate de diphényl-4,4 perhydrothiopyrano[2,3-c]pyrrole-(4aRS,7aRS) et de 1,70 $cm^3$ de triéthylamine dans 60 $cm^3$ de dichlorométhane sec, on ajoute par fractions de 0,5 g, 2,89 g de dicarbonate de ditert-butyle puis 0,15 g de diméthylamino-4 pyridine. On agite 20 heures à 20°C puis on lave la solution réactionnelle par 2 fois 100 $cm^3$ de solution aqueuse d'acide citrique de pH 4, par 100 $cm^3$ d'eau, sèche sur sulfate de magnésium, concentre à sec à 35°C sous pression réduite (2,7 kPa). Le résidu est cristallisé dans de l'éther éthylique, les cristaux sont essorés, et séchés. On obtient 4,27 g de diphényl-4,4 tertiobutyloxycarbonyl-6 perhydrothiopyrano[2,3-c]pyrrole-(4aRS,7aRS) sous la forme de cristaux roses fondant à 162°C.

Le diphényl-4,4 oxyde-1 perhydrothiopyrano[2,3-c]pyrrole-(1RS,4aRS, 7aRS) peut être aussi préparé de la façon suivante:

A une solution refroidie à -3°C de 25 g de diphényl-4,4 perhydrothiopyrano[2,3-c]pyrrole(4aRS,7aRS) dans 500 $cm^3$ de dichlorométhane et 100 $cm^3$ de méthanol, on ajoute en 40 minutes une solution de 15,4 g d'acide chloro-3 peroxybenzoïque (à 85%) dans 400 $cm^3$ de dichlorométhane. Après une heure d'agitation à -3°C, le mélange réactionnel est lavé par 200 $cm^3$ d'une solution aqueuse d'hydrogénocarbonate de potassium à 10% et 100 $cm^3$ de cette même solution, puis séché sur sulfate de magnésium, et concentré à sec à 40°C sous pression réduite (2,7 kPa). Le résidu est cristallisé dans 50 $cm^3$ d'acétate d'éthyle, les cristaux sont repris par 200 $cm^3$ de dichlorométhane, la solution obtenue est lavée par 75 $cm^3$ d'une solution aqueuse d'hydroxyde de sodium 1N puis séchée sur sulfate de magnésium et concentrée à sec. Le résidu est cristallisé dans 30 $cm^3$d'acétate d'éthyle, les cristaux sont lavés par de l'acétate d'éthyle, essorés et séchés. On obtient 13,6 g de diphényl-4,4 oxyde-1 perhydro-thiopyrano[2,3-c]pyrrole(1RS,4aRS,7aRS) sous la forme de cristaux blancs fondant à 174°C.

Exemple 3

A 7,15 g de diphényl-4,4 oxyde-1 perhydrothiopyrano[2,3-c]pyrrole-(1RS,4aRS,7aRS) on ajoute 3,5 g d'acide mandélique-(S) et 90 $cm^3$ d'un mélange d'acétonitrile et d'eau (99/1 en volumes). Après agitation la solution résultante est laissée pendant 48 heures à température ambiante. Les cristaux obtenus sont essorés, lavés par avec le mélange acétonitrile-eau, puis séchés. Les cristaux sont repris par 200 $cm^3$ du mélange acétonitrile-eau bouillant et après filtration à chaud, la solution obtenue est laissée 5 heures à température ambiante. Les cristaux sont essorés, lavés 2 fois par 10 $cm^3$ d'acétonitrile, puis séchés. On obtient 1,5 g de mandélate-(S) de diphényl-4,4 oxyde-1 perhydrothiopyrano[2,3-c]pyrrole-(1R*,4aR*,7aR*) $[\alpha]^{20}D$ = -228°, (c = 0,44; acide acétique). Le filtrat est laissé 20 heures à température ambiante, les cristaux obtenus sont essorés, lavés 2 fois par 5 $cm^3$ d'acétonitrile, puis séchés. On obtient 0,62 g de mandélate-(S) de diphényl-4,4 oxyde-1 perhydrothiopyrano [2,3-c]pyrrole-(1R*,4aR*,7aR*); $[\alpha]^{20}D$ = -230°, (c = 0,45; acide acétique).

A 2,06 g de mandélate-(S) de diphényl-4,4 oxyde-1 perhydrothiopyrano[2,3-c]pyrrole-(1R*,4aR*,7aR*) on ajoute 40 $cm^3$ de dichlorométhane et 7,0 $cm^3$ de soude aqueuse 1N. Le mélange est agité quelques minutes après dissolution du produit de départ, la phase organique est séchée sur sulfate de magnésium, concentrée à sec sous pression réduite (2,7 kPa). Le résidu est délité dans un mélange d'acétate d'éthyle et d'éther éthylique, le solide est lavé par de l'oxyde de diisopropyle, et séché. On obtient 1,14 g de (-)-diphényl-4,4 oxyde-1 perhydrothiopyrano [2,3-c]pyrrole-(1R*,4aR*,7aR*) sous la forme d'un solide blanc fondant à 192°C. $[\alpha]^{20}D$ = -405°, (c = 0,46; acide acétique).

Exemple 4

A 32,3 g de diphényl-4,4 oxyde-1 perhydrothiopyrano[2,3-c] pyrrole-(1RS,4aRS,7aRS) on ajoute 15,8 g d'acide (+)-mandélique-(S) et 750 $cm^3$ d'un mélange d'acétonitrile et d'eau (99/1 en volumes) puis 5,0 $cm^3$ d'eau. Après tièdissement, la solution obtenue est laissée pendant 48 heures à température ambiante. La suspension cristalline est filtrée, le filtrat concentré à sec donne une meringue que l'on reprend par 200 $cm^3$ du mélange acétonitrile-eau bouillant. La solution obtenue est laissée 20 heures environ à température ambiante. Les cristaux sont essorés, lavés par de l'acétonitrile, séchés, puis repris à nouveau par 200 $cm^3$ d'un mélange acétonitrile-eau (98/2 en volumes). La solution résultante est laissée 20 heures environ à température ambiante. Les cristaux sont essorés, séchés. On obtient 9,4 g de (+)-mandélate-(S) de diphényl-4,4 oxyde-1 perhydrothiopyrano[2,3-c]pyrrole-(1R*,4aR*, 7aR*); $[\alpha]^{20}D$ = +337°, (c = 0,45; acide acétique).

A 9,2 g de (+)-mandélate-(S) de diphényl-4,4 oxyde-1 perhydrothiopyrano[2,3-c]pyrrole-(1R*,4aR*,7aR*) on ajoute 100 $cm^3$ de dichlorométhane et 30 $cm^3$ de soude aqueuse 1N. Le mélange est agité pendant 10 minutes, la phase organique est séchée sur sulfate de magnésium, concentrée à sec sous pression réduite (2,7 kPa). Le résidu est délité dans un mélange d'acétate d'éthyle et d'oxyde de diisopropyle, le solide est lavé par de l'oxyde de diisopropyle et séché. On obtient 5,6 g de(+)-diphényl-4,4 oxyde-1 perhydrothiopyrano[2,3-c]pyrrole-(1R*,4aR*,7aR*) sous la forme d'un solide blanc fondant à 198°C. $[\alpha]^{20}D$ = +434°, (c = 0,45; acide acétique).

Exemple 5

On traite 0,58 g de diphényl-4,4 vinyloxycarbonyl-6 perhydrothiopyrano [2,3-c]pyrrole dioxyde-1,1-(4aRS,7aRS) par 25 $cm^3$ d'une solution 5,7 N d'acide chlohydrique dans le dioxane sec pendant 30 minutes en tiédissant. La solution réactionnelle est concentrée à sec sous pression réduite (2,7 kPa) à 50°C. Le résidu est repris par 15 $cm^3$ d'éthanol, la solution obtenue est portée au reflux pendant 30 minutes, puis est concentrée à sec. Le solide obtenu est lavé par de l'éther éthylique, essoré, et séché. On obtient 0,46 g de chlorhydrate de diphényl-4,4 perhydrothiopyrano[2,3-c]pyrrole dioxyde-1,1-(4aRS,7aRS), sous la forme d'un solide blanc.

Spectre infra-rouge (bandes caractéristiques, $cm^{-1}$): 3055, 3030, 2970, 2935, 2825, 2300, 1600, 1495, 1462, 1335, 1315, 1300, 1140, 1120, 770, 760, 710, 595, 505.

Spectre RMN du proton (DMSO-$d_6$ + $CF_3CCOD$, signaux principaux): 3,84 (ab, 2H, $CH_2$ en 7); 4,0 (mt, 1H, H en 4a); 4,27 (mt, 1H, H en 7a); 7,1 à 7,6 (mt, 10H, aromatigues).

Le diphényl-4,4 vinyloxycarbonyl-6 perhydrothiothiopyrano[2,3-c] pyrrole dioxyde-1,1-(4aRS,7aRS) peut être préparé de la façon suivante:

A une solution de 0,7 g benzyl-6 diphényl-4,4 perhydrothiopyrano[2,3-c]pyrrole dioxyde-1,1-(4aRS,7aRS) dans 10 $cm^3$ de dichloro-1,2 éthane on ajoute 0,16 $cm^3$ de chloroformiate de vinyle. Le mélange est porté au reflux pendant 2 heures puis concentré à sec sous pression réduite (2,7 kPa), à 50°C. Le solide cristallin est lavé par de l'éther éthylique, essoré puis séché. On obtient 0,58 g de diphényl-4,4 vinyloxycarbonyl-6 perhydrothiopyrano[2,3-c]pyrrole dioxyde-1,1-(4aRS,7aRS), sous la forme de cristaux blancs.

Spectre infra-rouge (bandes caractéristigues, $cm^{-1}$): 3080, 3055, 3025, 2990, 2970, 2925, 2885, 1715, 1645, 1595, 1580, 1495, 1415, 1330, 1300, 1150, 1140, 1125, 945, 865, 755, 700, 510.

Spectre RMN du proton (DMSO-$d_6$ + $CF_3COOD$, signaux principaux): 2,5 à 3,45 (mt, 6H, $CH_2$ en 5, en 2 et en 3); 3,8 à 4,2 (mt, 4H, $CH_2$ en 7, H en 4a et H en 7a); 4,46 et 4,72 (2d large, J=6 et J=14, 2x1H, CH=$\underline{CH_2}$); 7,0 (dd, J=14 et 6, 1H, O$\underline{CH}$=); 7,1 à 7,6 (mt, 10H, aromatiques).

Le benzyl-6 diphényl-4,4 perhydrothiopyrano[2,3-c]pyrrole dioxyde-1,1-(4aRS,7aRS) peut être préparé de la façon suivante:

A une solution de 1,3 g de dihydro-3,4 diphényl-4,4 dioxyde-1,1 2H-thiapyranne et de 1,75 $cm^3$ de N-butoxyméthyl N-triméthylsilylméthyl benzylamine dans 12 $cm^3$ de dichlorométhane anhydre, on ajoute 2 gouttes d'acide trifluoroacétique et agite pendant 30 minutes à 30°C. 1,75 $cm^3$ de N-butoxyméthyl N-triméthylsilylméthyl benzylamine et 2 gouttes d'acide trifluoroacétique sont à nouveau ajoutés et le mélange est agité pendant 2 heures à 35°C. Cette dernière opération est répétée une nouvelle fois, et après 1 heure d'agitation, on ajoute 1 g de carbonate de potassium. La suspension est filtrée, le filtrat concentré à sec sous pression réduite (2,7 kPa). Le résidu est chromatographié sur colonne de gel de silice (granulométrie 0,04-0,06 mm , diamètre 3,2 cm , hauteur 35 cm) , en éluant sous une pression de 0,5 bar d'azote par un mélange de cyclohexane et d'acétate d'éthyle (80/20 en volumes) et en recueillant des fractions de 30 $cm^3$. Les fractions 20 à 28 sont réunies et concentrées à sec sous pression réduite (2,7 kPa). On obtient 0,7 g de benzyl-6 diphényl-4,4 perhydrothiopyrano[2,3-c] pyrrole dioxyde-1,1-(4aRS,7aRS), sous la forme de cristaux blancs fondant à 186°C.

Le dihydro-3,4 diphényl-4,4 dioxyde-1,1 2H-thiapyranne peut être préparé de la façon suivante:

A une solution de 1,47 g de dihydro-3,4 diphényl-4,4 oxyde-1 2H-thiapyranne dans 15 $cm^3$ de dichlorométhane sec, on ajoute une solution de 1,12 g d'acide chloro-3 peroxybenzoïque (à 85%) dans 25 $cm^3$de dichlorométhane sec. Après 20 heures d'agitation à 20°C, le mélange réactionnel est lavé par 50 $cm^3$ d'une solution aqueuse à 10% de thiosulfate de sodium puis par 50 $cm^3$ de solution aqueuse saturée d'hydrogénocarbonate de sodium. La phase organique est séchée sur sulfate de magnésium, concentrée à sec ous pression réduite (2,7 kPa).Le résidu cristallin est lavé par de l'ether éthylique, essoré, séché sous pression réduite (2,7 kPa). On obtient 1,3 g de dihydro-3,4 diphényl-4,4 dioxyde-1,1 2H-thiapyranne sous la forme de cristaux blancs fondant à 166°C.

Le dihydro-3,4 diphényl-4,4 oxyde-1 2H-thiapyranne peut être préparé de la façon suivante:

En opérant comme ci-dessus, à partir de 2,05 g de dihydro-3,4 diphényl-4,4 2H-thiapyranne et de 1,67 g d'acide chloro-3 peroxybenzoïque (à 85%), on obtient 1,9 g de dihydro-3,4 diphényl-4,4 oxyde-1 2H-thiapyranne, sous la forme d'un solide blanc fondant à 130°C.

Le dihydro-3,4 diphényl-4,4 2H-thiapyranne peut être préparé de la façon suivante:

A une suspension de 2,7 g de diphényl-4,4 oxyde-1 tétrahydrothiapyranne dans 30 $cm^3$ de toluène anhydre, on ajoute 3,95 $cm^3$ d'anhydride acétique. Le mélange est porté au reflux pendant 20 heures et concentré à sec à 60°C sous pression réduite (2,7 kPa puis 0,13 kPa). Le résidu huileux est cristallisé dans de l'oxyde de diisopropyle, les cristaux sont essorés et séchés. On obtient 2,1 g de dihydro-3,4 diphényl-4,4 2H-thiapyranne sous la forme de cristaux blancs, fondant à 78°C.

Le diphényl-4,4 oxyde-1 tétrahydrothiapyranne peut être préparé de la façon suivante:

A une solution refroidie à 0°C de 25,4 g de diphényl-4,4 tétrahydrothiapyranne dans 130 $cm^3$ de dichlorométhane on ajoute en 40 minutes une solution de 20,3 g d'acide chloro-3 peroxybenzoïque (à 85%) dans 300 $cm^3$ de dichlorométhane. Après 2 heures d'agitation à 0°C, on ajoute au mélange 250 $cm^3$ d'une solution aqueuse d'hydrogénocarbonate de potassium à 5% puis agite pendant 15 minutes. La phase organique est lavée à nouveau par 250 $cm^3$ de la solution d'hydrogénocarbonate de potassium, puis est séchée sur sulfate de magnésium, concentrée à sec (après contrôle de l'absence de peroxydes), sous pression réduite (2,7 kPa). On obtient 26,9 g de diphényl-4,4 oxyde-1 tétrahydrothiapyranne, sous la forme d'un solide blanc fondant à 122°C.

Le diphényl-4,4 tétrahydrothiapyranne peut être préparé de la façon suivante:

A une suspension de 140,8 g de diphényl-3,3 bis méthanesulfonyloxy-1,5 pentane dans 1400 $cm^3$ de butanol-1 on ajoute 100 g de sulfure de sodium nonahydraté. Le mélange est chauffé au reflux pendant 2 heures puis refroidi à 20°C environ, additionné ensuite de 1000 $cm^3$ d'eau, de 500 $cm^3$ d'acétate d'éthyle, de 500 $cm^3$ de dichlorométhane. Après agitation, la phase organique est séparée, lavée successivement par 1000 $cm^3$ d'eau, 500 $cm^3$ d'acide chlorhydrique 1N, 500 $cm^3$ de solution aqueuse saturée d'hydrogénocarbonate de sodium, 1000 $cm^3$ d'eau, puis séchée sur sulfate de magnésium et concentrée à sec à 60°C sous pression réduite (2,7 kPa). Le résidu est cristallisé dans de l'acétate d'éthyle, les cristaux sont lavés par de l'oxyde de diisopropyle, essorés, et séchés. On obtient 76 g de diphényl-4,4 tétrahydrothiapyranne sous la forme de cristaux blancs fondant à 134°C.

Le diphényl-3,3 bis méthanesulfonyloxy-1,5 pentane peut être préparé de la façon suivante:

A une solution refroidie à -20°C de 95 g de diphényl-3,3 pentane diol-1,5 (préparé selon P. EILBRACHT et coll. Chem. Ber. 118, 825-839 (1985)) dans 950 $cm^3$ de dichlorométhane et de 113 $cm^3$ de triéthylamine, on ajoute en 10 minutes une solution de 62 $cm^3$ de chlorure de méthanesulfonyle dans 100 $cm^3$ de dichlorométhane. Après 2 heures d'agitation à 20°C, le mélange réactionnel est lavé par 2 fois 500 $cm^3$ d'eau, la phase organique est séchée sur sulfate de magnésium, concentrée à sec sous pression réduite (2,7 kPa). Le résidu huileux est cristallisé dans l'éther éthylique, les cristaux sont lavés par de l'éther éthylique, essorés et séchés. On obtient 140 g de diphényl-3,3 bis méthanesulfonyloxy-1,5 pentane sous la forme de cristaux blancs fondant à 99°C.

Les produits selon l'invention peuvent conduire aux dérivés de thiopyranopyrrole de formule générale (IX) en opérant comme dans les exemples suivants :

Exemple d'utilisation 1

A une solution de 1,16 g d'acide diméthylamino-2 phénylacétique dans 20 $cm^3$ de dichlorométhane sec, on ajoute 1,18 g de N,N'-carbonyldiimidazole. On agite 30 minutes à +5°C puis on ajoute une solution de 2,0 g de chlorhydrate de diphényl-4,4 perhydrothiopyrano[2,3-c]pyrrole-(4aRS,7aRS) et de 1,83 $cm^3$ de triéthylamine dans 20 $cm^3$ de dichlorométhane. Le mélange réactionnel est agité pendant 1 heure à 20°C puis est lavé 2 fois par 50 $cm^3$ d'eau et séché sur sulfate de magnésium. La solution est filtrée, concentrée à sec sous pression réduite (2,7 kPa). L'huile obtenue est chromatographiée sur une colonne de gel de silice (0,04 mm-0,06 mm, diamètre 2,8 cm, hauteur 26 cm), en éluant sous une pression de 0,6 bar d'azote par un mélange de cyclohexane et d'acétate d'éthyle (60/40 en volumes), en recueillant des fractions de 60 $cm^3$. Les fractions 6 à 20 sont réunies et concentrées à sec sous pression réduite (2,7 kPa), le résidu est cristallisé dans un mélange d'acétonitrile et d'oxyde de diisopropyle. Les cristaux sont essorés, séchés. On obtient 2,16 g de [(diméthylamino-2 phényl)acétyl]-6 diphényl-4,4 perhydrothiopyrano [2,3-c]pyrrole-(4aRS,7aRS) sous la forme d'un solide blanc fondant à 163°C.

Exemple d'utilisation 2

En opérant comme à l'exemple d'utilisation 1 à partir de 1,85 g de bromhydrate d'acide [(pyrrolidinyl-1)-2 phényl]acétique, et de 2,0 g de chlorhydrate diphényl-4,4 perhydrothiopyrano[2,3-c]pyrrole-(4aRS,7aRS), on obtient 0,90 g de {[(pyrrolidinyl-1)-2 phényl] acétyl}-6 diphényl-4,4 perhydro-thiopyrano[2,3-c]pyrrole-(4aRS,7aRS), sous la forme de cristaux blancs fondant à 166°C.

Exemple d'utilisation 3

A une solution refroidie à 0°C de 2,63 g de chlorhydrate de diphényl-4,4 perhydrothiopyrano[2,1-c]pyrrole-(4aRS,7aRS) et de 2,42 $cm^3$ de triéthylamine dans 25 $cm^3$ de dichlorométhane, on ajoute en 5 minutes une solution de 1,15 $cm^3$ de chlorure de phénylacétyle dans 25 $cm^3$ de dichlorométhane. Après agitation une heure à 0°C et une heure à 20°C, on ajoute 20 $cm^3$ de dichlorométhane. Le mélange réactionnel est lavé deux fois par 100 $cm^3$ d'eau distillée, séché sur sulfate de magnésium, puis concentré à sec sous pression réduite (2,7 kPa). L'huile obtenue est chromatographiée sur une colonne de gel de silice (0,04-0,06 mm, diamètre 3,5 cm, hauteur 26 cm), en éluant sous une pression d'azote de 0,4 bar par un mélange de cyclohexane et d'acétate d'éthyle (80/20 en volumes), en recueillant des fractions de 125 $cm^3$. Les fractions 19 à 26 sont réunies et concentrées à sec sous pression réduite (2,7 kPa). On obtient 0,87 g de phénylacétyl-6 diphényl-4,4 perhydrothiopyrano[2,3-c]pyrrole-(4aRS,7aRS), sous la forme d'un solide blanc fondant à 210°C.

Exemple d'utilisation 4

A une solution de 0,92 g d'hydroxy-2 phényl acétique dans 30 $cm^3$ de dichlorométhane sec, on ajoute 0,58 $cm^3$ de chloroformiate d'éthyle. Après 15 minutes d'agitation à 20°C, on refroidit le mélange à -15°C et ajoute 0,85 $cm^3$ de triéthylamine. Après 2 heures d'agitation à -15°C, on ajoute en 20 minutes une suspension de 2 g de chlorhydrate de diphényl-4,4 perhydrothiopyrano[2,3-c]pyrrole-(4aRS,7aRS) et de 1,70 $cm^3$ de triéthylamine dans 30 $cm^3$ de dichlorométhane. Après agitation pendant 20 heures à 20°C, le mélange réactionnel est lavé par 50 $cm^3$d'acide chlorhydrique 1N, 50 $cm^3$ de solution aqueuse saturée de chlorure de sodium puis séché sur sulfate de magnésium, et concentré à sec sous pression réduite (2,7 kPa). Le résidu est cristallisé dans 20 $cm^3$ de dichlorométhane, les cristaux sont lavés par de l'oxyde de diisopropyle, essorés et séchés sous pression réduite (2,7 kPa). On obtient 1,02 g de diphényl-4,4 [(hydroxy-2 phényl)acétyl]-6 perhydrothiopyrano[2,3-c]pyrrole-(4aRS,7aRS), sous la forme de cristaux blancs fondant à 248°C.

Exemple d'utilisation 5

A une solution refroidie à 0°C de 1,16 g d'acide méthoxy-2 phénylacétique dans 20 $cm^3$ de dichlorométhane sec, on ajoute 1,13 g de N,N'-carbonyldiimidazole. On agite 40 minutes à 0°C puis on ajoute une solution de 2,15 g de chlorhydrate de diphényl-4,4 perhydrothiopyrano[2,3-c]pyrrole-(4aRS,7aRS) et de 0,9 $cm^3$ de triéthylamine dans 20 $cm^3$ de dichlorométhane. Le mélange réactionnel est agité pendant une heure à 0°C puis est lavé par une solution aqueuse saturée d'hydrogénocarbonate de sodium. La phase organique est séchée sur sulfate de magnésium, concentrée à sec sous pression réduite (2,7 kPa). Le solide obtenu est lavé par 30 $cm^3$ d'éther éthylique, 30 $cm^3$ d'oxyde de diisopropyle, puis est séché sous pression réduite (2,7 kPa). On obtient 2,66 g de diphényl-4,4 [(méthoxy-2 phényl)acétyl]-6 perhydrothiopyrano[2,3-c]pyrrole-(4aRS,7aRS), sous la forme d'un solide blanc fondant à 172°C.

Exemple d'utilisation 6

Le [(méthoxy-2 phényl)-2 propionyl-(S)]-6 diphényl-4,4 perhydrothiopyrano[2,3-c]pyrrole-mélange des formes (4aR,7aR) et (4aS,7aS) peut être préparé en opérant comme décrit dans l'exemple 5, à partir de 0,89 g d'acide (méthoxy-2 phényl)-2 propionique-(S) et de chlorhydrate de diphényl-4,4 perhydrothiopyrano[2,3-c]pyrrole-(4aRS,7aRS). On obtient 1,46 g de [(méthoxy-2 phényl)-2 propionyl -(S)]-6 diphényl-4,4 perhydrothiopyrano[2,3-c]pyrrole-mélange des formes (4aR,7aR) et (4aS,7aS) sous la forme d'une meringue blanche.

Première forme :

Spectre infra-rouge (bandes caractéristiques, $cm^{-1}$): 3095, 3055, 3025, 2950, 2930, 2875, 2835, 1630 1595, 1490, 1565, 1425, 1240, 1030, 750, 700.

Spectre RMN du proton (DMSO-$d_6$ + $CF_3COOD$, à température ambiante, on observe le mélange des deux rotamères; signaux caractéristiques ): $^{1,15}$ et 1,20 (2d, J=7,5, 3H, $CH_3$); 2,1-2,9 (mt, 5H, 2 $CH_2$ en 5 et 3 + H en 4a); 3,36 et 3,8 (2s, 3H, $OCH_3$); 6,7 à 7,4 (mt, 14H, aromatiques) .

Deuxième forme :

Spectre infra-rouge (bandes caractéristiques, $cm^{-1}$): 3095, 3060, 3025, 2960, 2930, 2870, 2835, 1640, 1600, 1495, 1465, 1425, 1240, 1035, 755, 700.

Spectre RMN du proton (DMSO-$d_6$ + $CF_3COOD$, à température ambiante, on observe le mélange des deux rotamères; signaux caractéristiques): 1,1 et 1,18 (2d, J=7,5, 3H, $CH_3$); 2,1-2,35 (mt, 2H, $CH_2$ en 3); 2,35-3,10 (mt, 3H, $CH_2$ en 5 + H en 4a); 3,6 et 3,8 (2s, 3H, $OCH_3$); 3,95 et 4,02 (mt, 1H, H en 7a); 6,7 à 7,4 (mt, 14H, aromatiques).

L'acide (méthoxy-2 phényl)-2 propionique-(S) peut être obtenu de la manière suivante :

L'acide (méthoxy-2 phényl)-2 propionique-(S) peut être préparé, par analogie avec les méthodes décrites par D.A Evans et coll., Tetrahedron, 44, 5525,(1988), selon le mode opératoire suivant:

A une solution refroidie à +5°C de 4,1 g de méthyl-4 phényl-5 [(méthoxy-2 phényl)-2-(S)-propionyl]-3 oxazolidinone-2-(4S,5S) dans 60 $cm^3$ de tétrahydrofuranne et 30 $cm^3$ d'eau, on ajoute 1,52 g d'hydroxyde de lithium. Le mélange réactionnel est agité 3 heures à cette tempétature, puis, après retour à température ambiante, on rajoute de l'acétate d'éthyle, on décante, la phase aqueuse est acidifiée par une solution aqueuse d'acide chlorhydrique 1N, extraite par de l'acétate d'éthyle, la phase organique est séchée sur sulfate de magnésium, et concentrée à sec sous pression réduite (2,7 kPa). Le solide obtenu est recristallisé dans l'hexane, essoré et séché. On obtient 0,4 g d'acide (méthoxy-2 phényl)-2 propionique-(S) sous la forme de cristaux blancs fondant à 102°C. $[a]_D^{20}$ = +84,6°; (c=1; $CHCl_3$).

La méthyl-4 phényl-5 [(méthoxy-2 phényl)-2-(S)-propionyl]-3 oxazolidinone-2-(4S,5S) peut être obtenue de la manière suivante :

A une solution refroidie à -50°C de 10 g de méthyl-4 phényl-5 [(méthoxy-2 phényl)-acétyl]-3 oxazolidinone-2-(4S,5S) dans 150 $cm^3$ de tétrahydrofuranne on ajoute 19,1 g d'hexaméthyl-1,1,1,3,3,3 disilazanate de sodium, on agite 45 minutes à cette température, puis on ajoute 7,72 $cm^3$ d'iodure de méthyle. Le mélange réactionnel est ensuite agité 15 heures à température ambiante, puis dilué par de l'acétate d'éthyle, lavé par 50 $cm^3$ d'eau, puis par 50 $cm^3$ d'une solution aqueuse saturée de chlorure de sodium, séché sur sulfate de magnésium et concentré à sec sous pression réduite (2,7 kPa). Le résidu obtenu est cristallisé dans l'oxyde d'isopropyle, essoré et séché. On obtient 4,2 g de méthyl-4 phényl-5 [(méthoxy-2 phényl)-2-(S)-propionyl]-3 oxazolidinone-2- (4S,5S) sous la forme d'un solide blanc.

La méthyl-4 phényl-5 (méthoxy-2 phénylacétyl)-3 oxazolidinone-2-(4S,5S) peut être obtenue de la manière suivante :

A une suspension de 1,89 g d'hydrure de sodium (dispersion à 80% dans la vaseline) dans 200 $cm^3$ de tétrahydrofuranne sec on ajoute à température ambiante 9,38 g d'acide méthoxy-2 phénylacétique. On refroidit cette suspension à -30°C, on ajoute 7,77 $cm^3$ de chlorure de pivaloyle, puis on ajoute enfin une solution refroidie à -78°C obtenue en additionnant une solution de 35,27 $cm^3$ de butyllithium 1,6 M dans l'hexane à une solution refroidie à -78°C de 10 g de méthyl-4 phényl-5 oxazolidinone-2-(4S,5S) dans 200 $cm^3$ de tétrahydrofuranne sec. Le mélange réactionnel est agité 45 minutes à -30°C, puis après retour à température ambiante, on ajoute 200 $cm^3$ d'une solution aqueuse saturée de chlorure d'ammonium, puis 500 $cm^3$ d'acétate d'éthyle ; aprés décantation la phase organique est lavée deux fois par 100 $cm^3$ d'eau, puis deux fois par 100 $cm^3$ d'une solution aqueuse saturée de chlorure de sodium; séchée sur sulfate de magnésium et concentrée à sec sous pression réduite (2,7 kPa). Le résidu est chromatographié sur colonne de gel de silice (granulométrie 0,04-0,06 mm, diamètre 4,8 cm, hauteur 36 cm), en éluant sous une pression de 0,6 bar d'azote par un mélange de cyclohexane et d'acétate d'éthyle (85/15 puis 80/20 en volumes ) et en recueillant des fractions de 50 $cm^3$. Les fractions 14 à 31 sont réunies et concentrées à sec sous pression réduite (2,7 kPa). On obtient 13,6 g de méthyl-4 phényl-5 (méthoxy-2 phénylacétyl)-3 oxazolidinone-2-(4S,5S) sous la forme d'une huile jaune.

Exemple d'utilisation 7

En opérant selon le mode opératoire de l'exemple 8 ci-après, à partir de 1,82 g de diphényl-4,4 oxyde-1 perhydrothiopyrano[2,3-c]pyrrole-(1RS,4aRS,7aRS) et de 1,39 g d'acide ((diméthylamino-3 propoxy-2) phényl] acétique, on obtient 0,3 g de {[(diméthylamino-3 propoxy-2)phényl] acétyl}-6 diphényl-4,4 oxyde-1 perhydrothiopyrano[2,3-c]pyrrole-(1RS,4aRS,7aRS) sous la forme de cristaux blancs fondant à 150°C.

Exemple d'utilisation 8

A une solution refroidie à 0°C de 1,06 g de (-)-diphényl-4,4 oxyde-1 perhydrothiopyrano[2,3-c]pyrrole-(1R∗ ,4aR∗ ,7aR∗ ) et de 0,81 g d'acide [(diméthylamino-3 propoxy-2)phényl]-2 acétique dans 60 $cm^3$ de dichlorométhane sec, on ajoute 0,03 g d'hydrate d'hydroxybenzotriazole, puis 0,77 g de chlorhydrate de (diméthylamino-3 propyl)-1 éthyl-3 carbodiimide. Après 2 heures d'agitation à 0°C et 20 heures à 20°C, le mélange réactionnel est lavé par 20 $cm^3$ d'eau puis séché sur sulfate de magnésium, et concentré à sec sous pression réduite (2,7 kPa). Le résidu est chromatographié sur colonne de gel de silice (granulométrie 0,04-0,06 mm , diamètre 2,4 cm , hauteur 35 cm) , en éluant sous une pression de 0,6 bar d'azote par un mélange d'acétate d'éthyle, d'acide acétique et d'eau (60/10/10 en volumes) et en recueillant des fractions de 50 $cm^3$. Les fractions 8 à 19 sont réunies et concentrées à sec sous pression réduite (2,7 kPa). Le résidu est repris avec 60 $cm^3$ de dichlorométhane, la solution est lavée par 20 $cm^3$ de solution aqueuse d'hydroxyde de sodium 1N, séchée sur sulfate de magnésium, puis concentrée à sec. Le solide obtenu est recristallisé dans un mélange d'acétate d'éthyle et d'éther éthylique, les cristaux sont lavés par de l'oxyde de diisopropyle, essorés, et séchés. On obtient 0,99 g de (-)-{[(diméthylamino-3 propoxy-2)phényl]-2 acétyl}-6 diphényl-4,4 oxyde-1 perhydrothiopyrano[2,3-c]pyrrole-(1R∗ ,4aR∗ ,7aR∗) sous la forme d'un solide blanc fondant à 120°C. $[a]^{20}D$ = -323°; (c = 0,5; acide acétique).

Exemple d'utilisation 9

A une solution refroidie à +5°C de 0,69 g de diphényl-4,4 oxyde-1 perhydrothiopyrano[2,3-c]pyrrole-(1RS,4aRS,7aRS) et de 0,68 g d'acide {[(pyrrolidinyl-1)-3 propoxy-2]phényl}acétique dans 25 $cm^3$ de dichlorométhane sec, on ajoute 0,03 g d'hydrate d'hydroxybenzotriazole, puis une solution de 0,5 g de chlorhydrate de (diméthylamino-3 propyl)-1 éthyl-3 carbodiimide dans 20 $cm^3$ de dichlorométhane sec. Après 2 heures d'agitation à +5°C et 20 heures à 20°C, le mélange réactionnel est lavé 2 fois par 50 $cm^3$ d'eau distillée et séché sur sulfate de magnésium puis concentré à sec sous pression réduite (2,7 kPa). Le résidu obtenu est chromatographié sur colonne de gel de silice (granulométrie 0,04-0,06 mm , diamètre 2,4 cm , hauteur 32 cm), en éluant sous une pression de 0,8 bar d'azote par un mélange d'acétate d'éthyle, d'acide acétique, et d'eau (80/20/20 en volumes) et en recueillant des fractions de 25 $cm^3$. Les fractions 21 à 50 sont réunies et concentrées à sec sous pression réduite (2,7 kPa). Le résidu est cristallisé dans 8 $cm^3$ d'acétate d'éthyle, les cristaux sont lavés par de l'acétate d'éthyle et de l'oxyde de diisopropyle puis séchés. On obtient 0,45 g de {{[(pyrrolidinyl-1)-3 propoxy-2]phényl}acétyl}-6 diphényl-4,4 oxyde-1 perhydrothiopyrano[2,3-c]pyrrole-(1RS,4aRS, 7aRS) sous la forme de cristaux beiges fondant à 126°C.

Exemple d'utilisation 10

A une solution refroidie à 0°C environ de 1,43 g de (-)-diphényl-4,4 oxyde-1 perhydrothiopyrano[2,3-c]pyrrole-(1R∗,4aR∗,7aR∗), et de 0,83 g d'acide (méthoxy-2 phényl)-2 propionique-(S) dans 100 $cm^3$ de dichlorométhane sec, on ajoute 0,06 g d'hydrate d'hydroxybenzotriazole et 1,01 g de chlorhydrate de (diméthylamino-3 propyl)-1 éthyl-3 carbodiimide. Après 2 heures d'agitation à 0°C et 2 heures à 20°C, le mélange réactionnel est lavé par 50 $cm^3$ d'eau puis séché sur sulfate de magnésium, et concentré à sec sous pression réduite (2,7 kPa). Le résidu est cristallisé dans de l'acétonitrile, les cristaux sont essorés, lavés plusieurs fois par de l'éther éthylique, puis séchés.

On obtient 1,56 g de (-)-(méthoxy-2 phényl)-2 propionyl-(S)]-6 diphényl-4,4 oxyde-1 perhydrothiopyrano[2,3-c]pyrrole-(1R∗,4aR∗,7aR∗) sous la forme de cristaux blancs fondant à 170°C. $[\alpha]^{20}D$ = -316°; (c = 0,50; acide acétique).

Exemple d'utilisation 11

En opérant comme décrit précédemment dans l'exemple 1, à partir de 0,49 g d'acide diméthylamino-2 phénylacétique, de 0,50 g de N,N'-carbonyldiimidazole, de 0,70 $cm^3$de triéthylamine et de 1,0 g de chlorhydrate

de diphényl-4,4 perhydrothiopyrano[2,3-c]pyrrole dioxyde-1,1-(4aRS,7aRS), on obtient 0,55 g de [(diméthylamino-2 phényl)-6 diphényl-4,4 perhydrothiopyrano[2,3-c]pyrrole dioxyde-1,1-(4aRS,7aRS), sous la forme de cristaux blancs fondant à 226°C.

Exemple d'utilisation 12

A une suspension refroidie à 0°C de 0,46 g de chlorhydrate de diphényl-4,4 perhydrothiopyrano[2,3-c]pyrrole dioxyde-1,1-(4aRS,7aRS) dans 10 $cm^3$ de dichlorométhane, on ajoute 0,35 $cm^3$ de triéthylamine, et une solution de 0,17 $cm^3$ de chlorure de phénylacétyle dans 5 $cm^3$ de dichlorométhane. Après 1 heure d'agitation à 0°C puis 1 heure à 20°C, le mélange réactionnel est dilué avec 10 $cm^3$ de dichlorométhane, lavé 2 fois par 30 $cm^3$ d'eau distillée, séché sur sulfate de magnésium et concentré à sec sous pression réduite (2,7 kPa). L'huile obtenue est cristallisée dans 30 $cm^3$ d'éther éthylique, les cristaux sont essorés, et séchés. On obtient 0,50 g de diphényl-4,4 phénylacétyl-6 perhydrothiopyrano[2,3-c]pyrrole dioxyde-1,1-(4aRS,7aRS), sous la forme de cristaux blancs.

Spectre infra-rouge (bandes caractéristiques, $cm^{-1}$): 3050, 3025, 2970, 2930, 1880, 1630, 1595, 1495, 1455, 1425, 1330, 1305, 1140, 1120, 765, 755, 700, 510.

Spectre RMN du proton (DMSO-$d_6$ + $CF_3CCOD$, signaux principaux, à température ambiante on observe le mélange des deux rotamères): 2,48 (mt, 1H, $CH_2$ en 3); 2,8 (mt, 1H, 1H en 5); 3,39 et 3,65 (s et ab J=14, 2H, N-CO-$CH_2$); 6,9 à 7,6 (mt, 15H, aromatiques).

Exemple d'utilisation 13

A une suspension de 1,5 g de chlorhydrate de diphényl-4,4 perhydrothiopyrano[2,3-c]pyrroledioxyde-1,1-(4aRS,7aRS) et de 0,95 g de chlorhydrate d'éthoxy-1 imino-1 (méthoxy-2 phényl)-2 éthyle dans 15 $cm^3$ de dichloro-1,2 éthane, on ajoute goutte à goutte 1,16 $cm^3$ de triéthylamine. Après 20 heures d'agitation à 20°C, le mélange est additionné de 30 $cm^3$ de dichlorométhane puis lavé successivement par 100 $cm^3$ d'eau et 100 $cm^3$ de solution aqueuse de carbonate de potassium à 5%. La phase organique est séchée sur sulfate de magnésium, concentrée à sec sous pression réduite (2,7 kPa). Le résidu est cristallisé dans un mélange d'acétonitrile et d'oxyde de diisopropyle. Les cristaux sont lavés par de l'acétonitrile puis par de l'oxyde de diisopropyle, essorés, et séchés. On obtient 0,83 g d'imino-1 (méthoxy-2 phényl)-2 éthyl-6 diphényl-4,4 perhydrothiopyrano[2,3-c]pyrrole dioxyde-1,1-(4aRS,7aRS) sous la forme de cristaux blancs fondant à 240°C.

**Revendications**

**1 -** Nouveau dérivé de thiopyranopyrrole caractérisé en ce qu'il répond à la formule générale :

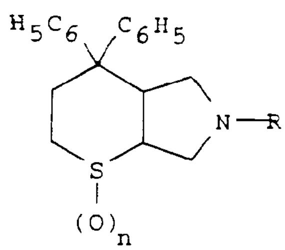

dans laquelle le symbole R représente un atome d'hydrogène, un radical allyle ou un radical de structure :

$$- C R_aR_bR_c$$

dans laquelle $R_a$ et $R_b$ sont des atomes d'hydrogène ou des radicaux phényle éventuellement substitués (par un atome d'halogène, un radical alcoyle, alcoyloxy ou nitro), et $R_c$ est défini comme $R_a$ et $R_b$ ou représente un radical alcoyle ou alcoyloxyalcoyle, l'un au moins de $R_a$, $R_b$ et $R_c$ étant un radical phényle substitué ou non, et le symbole n représente un nombre entier de 0 à 2, étant entendu que les radicaux alcoyle cités ci-dessus contiennent 1 à 4 atomes de carbone en chaîne droite ou ramifiée, ainsi que ses formes stéréoisomères et ses sels lorsqu'ils existent.

**2 -** Le diphényl-4,4 perhydrothiopyrano[2,3-c]pyrrole, ainsi que ses sels, sous ses formes stéréoisomères et leurs mélanges.

**3 -** Le diphényl-4,4 oxyde-1 perhydrothiopyrano[2,3-c]pyrrole, ainsi que ses sels, sous ses formes stéréoisomères et leurs mélanges.

**4 -** Le diphényl-4,4 perhydrothiopyrano[2,3-c]pyrrole dioxyde-1,1, ainsi que ses sels, sous ses formes stéréoisomères et leurs mélanges.

**5 -** Le diphényl-4,4 vinyloxycarbonyl-6 perhydrothiopyrano[2,3-c] pyrrole, sous ses formes stéréoisomères et leurs mélanges.

**6 -** Le diphényl-4,4 oxyde-1 tertiobutyloxycarbonyl-6 perhydrothiopyrano[2,3-c]pyrrole, sous ses formes stéréoisomères et leurs mélanges.

**7 -** Procédé de préparation d'un nouveau dérivé de thiopyranopyrrole selon la revendication 1, caractérisé en ce que l'on traite un dérivé de formule générale :

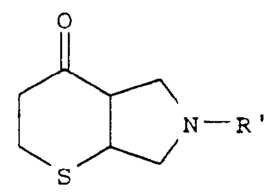

dans laquelle R′ est défini comme R dans la revendication 1 à l'exception de représenter l'atome d'hydrogène, successivement par un halogénure de phényl-magnésium, puis par le benzène en présence de tétrachlorure de zirconium, puis éventuellement élimine du radical protecteur R′ si l'on veut obtenir un produit pour lequel R est un atome d'hydrogène, et/ou, le cas échéant, oxyde le produit obtenu, pour obtenir un dérivé de thiopyranopyrrole pour lequel n = 1 ou 2, puis sépare éventuellement les stéréoisomères et transforme éventuellement le produit obtenu en un sel lorsqu'ils existent.

**8 -** Procédé de préparation d'un dérivé de thiopyranopyrrole selon la revendication 1, pour lequel le symbole n est égal à 1 ou 2, caractérisé en ce que l'on oxyde un dérivé de thiopyranopyrrole selon la revendication 1, pour lequel le symbole n est égal à 0, et dont le cas échéant la fonction amine est préalablement protégée, par toute méthode connue pour l'oxydation de sulfures en sulfoxydes ou en sulfones, qui n'altère pas le reste de la molécule, puis le cas échéant, si l'on veut obtenir un produit pour lequel R est l'atome d'hydrogène, élimine le radical protecteur, sépare éventuellement les stéréoisomères et transforme éventuellement le produit obtenu en un sel lorsqu'ils existent.

**9 -** Procédé de préparation d'un dérivé de thiopyranopyrrole selon la revendication 1, pour lequel le symbole n est égal à 2, caractérisé en ce que l'on effectue une réaction de cycloaddition d'un dérivé silylé de formule générale :

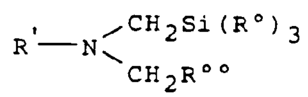

dans laquelle dans laquelle R′ est défini comme précédemment, $(R°)_3$ représente des radicaux alcoyle ou des radicaux alcoyle et phényle et R°° représente un radical alcoyloxy, cyano ou phénylthio, sur le dihydro-3,4 diphényl-4,4 di-oxyde-1,1 2H-thiapyranne de formule :

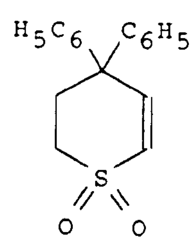

suivie éventuellement de l'élimination du radical protecteur R′, si l'on veut obtenir un dérivé de thiopyranopyrrole selon la revendication 1 pour lequel R est un atome d'hydrogène, puis sépare éventuellement les stéréoisomères et transforme éventuellement le produit obtenu en un sel lorsqu'ils existent.

**10 -** Utilisation d'un produit selon la revendication 1 pour la préparation d'un dérivé de thiopyranopyrrole de formule générale :

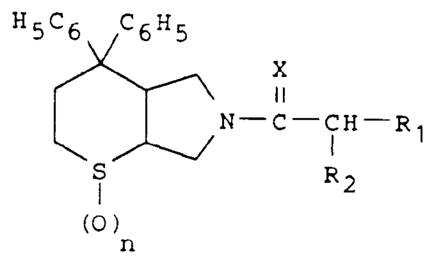

dans laquelle :

- le symbole n est un nombre entier de 0 à 2,
- le symbole X représente un atome d'oxygène, ou un radical NH,
- le symbole $R_1$ représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitués (par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy ou hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiényle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre, et
- le symbole $R_2$ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino, les radicaux alcoyle et acyle cités ci-dessus étant droits ou ramifiés et contenant 1 à 4 atomes de carbone, sous ses formes stéréoisomères ou leurs mélanges, ainsi que ses sels lorsqu'ils existent.

Office européen des brevets

# RAPPORT DE RECHERCHE EUROPEENNE

Numero de la demande

EP 92 40 1332

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A,D | US-A-4 042 707 (W.C. RIPKA)<br>* colonne 31, tableau I *<br>--- | 1 | C07D495/04<br>//(C07D495/04,<br>335:00,209:00) |
| A,D | EP-A-0 068 822 (ROHM AND HAAS CO.)<br>* abrégé *<br>--- | 1 | |
| A | AU-B-502 760 (SCIENCE UNION ET CIE.)<br>* revendications 1,2 *<br>--- | 1 | |
| A | EP-A-0 359 172 (SHIONOGI AND CO., LTD.)<br>* page 15, composé (II-11) *<br>--- | 1 | |
| A | EP-A-0 058 567 (WARNER-LAMBERT CO.)<br>* abrégé *<br>--- | 10 | |
| A | EP-A-0 093 805 (WARNER-LAMBERT CO.)<br>* abrégé *<br>----- | 10 | |
| | | | DOMAINES TECHNIQUES RECHERCHES (Int. Cl.5 ) |
| | | | C07D |

Le présent rapport a été établi pour toutes les revendications

| Lieu de la recherche | Date d'achèvement de la recherche | Examinateur |
|---|---|---|
| BERLIN | 21 AOUT 1992 | CHRISTIAN HASS |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)